(19) Europäisches Patentamt
European Patent Office
Office européen des brevets

(11) **EP 4 261 831 A1**

(12) **EUROPEAN PATENT APPLICATION**

(43) Date of publication:
**18.10.2023 Bulletin 2023/42**

(21) Application number: **23166704.9**

(22) Date of filing: **05.04.2023**

(51) International Patent Classification (IPC):
**G16C 20/50** (2019.01)  **G06N 3/00** (2023.01)
**G16C 20/70** (2019.01)

(52) Cooperative Patent Classification (CPC):
**G16C 20/50; G06N 3/00; G16C 20/70;** G16C 20/30

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.04.2022 EP 22168224**

(71) Applicant: **Bayer AG**
**51373 Leverkusen (DE)**

(72) Inventors:
• **Abdallah, Fuad**
  **42799 Leichlingen (DE)**
• **Hatz, Kathrin**
  **50674 Köln (DE)**

• **Görlitz, Linus**
  **51427 Bergisch Gladbach (DE)**
• **Weyßer, Fabian**
  **51371 Leverkusen (DE)**
• **O´Dowd, Bing Ashley Liang**
  **Düsseldorf, 40237 (US)**
• **Ernst, Matthias**
  **51063 Köln (DE)**
• **Grimbs, Sergio**
  **51373 Leverkusen (DE)**
• **Grimbs, Anne**
  **51373 Leverkusen (DE)**

(74) Representative: **BIP Patents**
**c/o Bayer Intellectual Property GmbH**
**Alfred-Nobel-Straße 50**
**40789 Monheim am Rhein (DE)**

(54) **PREDICTION OF CHEMICAL COMPOUNDS WITH DESIRED PROPERTIES**

(57)     Systems, methods, and computer programs disclosed herein relate to identifying chemical compounds having a desired property profile using a machine learning model.

Fig. 2

EP 4 261 831 A1

**Description**

FIELD

Systems, methods, and computer programs disclosed herein relate to identifying chemical compounds having a desired property profile using a machine learning model.

BACKGROUND

In the research and development departments of the chemical industry, new chemical compounds are constantly being synthesized and their properties characterized in order to develop new drugs, crop protection products and/or other products with improved properties.

The search for new compounds with improved properties can proceed in several phases. In a first phase, a large number of existing compounds can be screened for one or more properties (e.g., a biological activity). Compounds that exhibit the one or more properties (e.g., the biological activity) can then be the starting point for an optimization as so-called lead compound. The chemical structure of a lead compound can serve as a starting point for chemical modifications to improve efficacy, selectivity, toxicity, safety, solubility, and/or other properties.

Ways to speed up the process are constantly being sought, as the production of chemical compounds and their characterization cost both time and money.

There are approaches to first generate new chemical compounds in the computer (*in silico*) and calculate their properties, and then chemically synthesize and test promising candidates.

For example, R. Gomez-Bombarelli *et al.* disclose a method for automatic chemical design using a deep neural network (Automatic Chemical Design Using a Data-Driven Continuous Representation of Molecules, ACS Cent. Sci. 2018, 4, 268-276). The deep neural network comprises three units: an encoder, a decoder, and a predictor. The encoder converts the discrete representation of a molecule into a real-valued continuous vector, and the decoder converts such a continuous vector back to a discrete molecular representation. The predictor estimates chemical properties from the latent continuous vector representation of the molecule. The predictor is a multi-layer perceptron and thus latent continuous vectors are mapped to the chemical properties via a non-linear function. Such a non-linear mapping between the latent space representation and chemical properties makes it difficult to identify, decode and optimize molecules with desired properties since they could be located in multiple locations in the latent space.

S. Mohammadi *et al.* therefore propose to use linear units for property prediction (Penalized Variational Autoencoder for Molecular Design, DOI: 10.26434/chemrxiv.7977131.v2). The linear prediction unit can be inverted in order to map back to the latent space starting with a property vector, without prior knowledge of a molecular structure. However, the prediction of new chemical compounds based solely on a desired property profile works only to a very limited extent in practice. On one hand side, such completely new chemical compounds must first be synthesized; chemical building blocks for generating the new chemical structures are often not available. On the other hand side, the predicted chemical compounds very often do not perform as hoped (predicted) in testing.

In practice, it is more expedient to start from a lead compound and optimize it, since chemical building blocks are available for the lead structure and its derivatives, and the property profile of the lead compound is usually closer to the desired property profile than the property profile of completely newly predicted substances.

For the prediction of new chemical compounds based on existing lead compounds, neither R. Gomez-Bombarelli *et al.* nor S. Mohammadi *et al.* provide routinely feasible guidance. They propose to perturbate the latent vector of the lead compound in latent space in order to generate latent vectors of new compounds. The described procedure yields a comparatively large number of invalid compounds and/or compounds whose properties do not match the desired property profile.

SUMMARY

These and further problems are solved by the subject matter of the independent claims. Preferred embodiments can be found in the dependent claims, the present description, and the drawings.

Therefore, in a first aspect, the present disclosure provides a computer-implemented method, the method comprising:

- providing a trained machine learning model, the trained machine learning model comprising an encoder, a decoder, and a linear transformation unit,

  • wherein the encoder is configured and trained to convert a discrete molecular representation of a chemical compound into a vector in continuous latent space,

  • wherein the decoder is configured and trained to convert a vector in the continuous latent space into a discrete molecular representation of a chemical compound,

  • wherein the linear transformation unit is configured and trained to map a vector in the continuous latent space to a property vector representing a property profile,

- receiving a target property vector representing a target property profile,

- mapping the target property vector to the continuous latent space via the linear transformation unit, thereby determining a subset in the continuous latent space,

- receiving a molecular representation of a lead compound,

- converting the molecular representation of the lead compound to a vector representing the lead compound in the continuous latent space via the encoder,

- projecting the vector representing the lead compound in the continuous latent space onto the subset, thereby generating a first vector representing a first test compound in the continuous latent space,

- generating a discrete molecular representation of the first test compound using the decoder,

- inputting the discrete molecular representation of the first test compound into the encoder, thereby generating a second vector representing the first test compound in the continuous latent space,

- inputting the second vector representing the first test compound in the continuous latent space into the linear transformation unit, thereby generating a property vector representing a property profile of the first test compound,

- comparing the property profile of the first test compound with the target property profile,

- in case the property profile of the first test compound has a pre-defined similarity to the target property profile: outputting the discrete molecular representation of the first test compound and/or another representation of the first test compound.

In another aspect, the present disclosure provides a computer system comprising:

a processor; and
a memory storing an application program configured to perform, when executed by the processor, an operation, the operation comprising:

- providing a trained machine learning model, the trained machine learning model comprising an encoder, a decoder, and a linear transformation unit,

  • wherein the encoder is configured and trained to convert a discrete molecular representation of a chemical compound into a vector in continuous latent space,

  • wherein the decoder is configured and trained to convert a vector in the continuous latent space into a discrete molecular representation of a chemical compound,

  • wherein the linear transformation unit is configured and trained to map a vector in the continuous latent space to a property vector representing a property profile,

- receiving a target property vector representing a target property profile,

- mapping the target property vector to the continuous latent space via the linear transformation unit, thereby determining a subset in the continuous latent space,

- receiving a molecular representation of a lead compound,

- converting the molecular representation of the lead compound to a vector representing the lead compound in the continuous latent space via the encoder,

- projecting the vector representing the lead compound in the continuous latent space onto the subset, thereby generating a first vector representing a first test compound in the continuous latent space,

- generating a discrete molecular representation of the first test compound using the decoder,

- inputting the discrete molecular representation of the first test compound into the encoder, thereby generating a second vector representing the first test compound in the continuous latent space,

- inputting the second vector representing the first test compound in the continuous latent space into the linear transformation unit, thereby generating a property vector representing a property profile of the first test compound,

- comparing the property profile of the first test compound with the target property profile,

- in case the property profile of the first test compound has a pre-defined similarity to the target property profile: outputting the discrete molecular representation of the first test compound and/or another representation of the first test compound.

In another aspect, the present disclosure provides a non-transitory computer readable medium having stored thereon software instructions that, when executed by a processor of a computer system, cause the computer system to execute the following steps:

- providing a trained machine learning model, the trained machine learning model comprising an encoder, a decoder, and a linear transformation unit,

  • wherein the encoder is configured and trained to convert a discrete molecular representation of a chemical compound into a vector in continuous latent space,

  • wherein the decoder is configured and trained to convert a vector in the continuous latent space into a discrete molecular representation of a chemical compound,

  • wherein the linear transformation unit is configured and trained to map a vector in the continuous latent space to a property vector representing a property profile,

- receiving a target property vector representing a target property profile,

- mapping the target property vector to the continuous latent space via the linear transformation unit, thereby determining a subset in the continuous latent space,

- receiving a molecular representation of a lead compound,

- converting the molecular representation of the lead compound to a vector representing the lead compound in the continuous latent space via the encoder,

- projecting the vector representing the lead compound in the continuous latent space onto the subset, thereby generating a first vector representing a first test compound in the continuous latent space,

- generating a discrete molecular representation of the first test compound using the decoder,

- inputting the discrete molecular representation of the first test compound into the encoder, thereby generating a second vector representing the first test compound in the continuous latent space,

- inputting the second vector representing the first test compound in the continuous latent space into the linear transformation unit, thereby generating a property vector representing a property profile of the first test compound,

- comparing the property profile of the first test compound with the target property profile,

- in case the property profile of the first test compound has a pre-defined similarity to the target property profile: outputting the discrete molecular representation of the first test compound and/or another representation of the first test compound.

BRIEF DESCRIPTION OF THE DRAWINGS

Fig. 1 shows schematically an example of a machine learning model of the present disclosure.

Fig. 2 shows schematically an example of training a machine learning model of the present disclosure.

Fig. 3 shows schematically an example of projecting a vector representing a lead compound onto a subset of the continuous latent space representing a target property profile.

Fig. 4 (a) to Fig. 4 (i) show schematically an example of identifying a test compound using a trained machine learning model.

Fig. 5 illustrates a computer system according to some example implementations of the present disclosure in more detail.

Fig. 6 shows an embodiment of the computer-implemented method of predicting a test compound in the form of a flow chart.

DETAILED DESCRIPTION

The invention will be more particularly elucidated below without distinguishing between the aspects of the disclosure (method, computer system, computer-readable storage medium). On the contrary, the following elucidations are intended to apply analogously to all the aspects of the disclosure, irrespective of in which context (method, computer system, computer-readable storage medium) they occur.

If steps are stated in an order in the present description or in the claims, this does not necessarily mean that the disclosure is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides means for predicting chemical compounds with desired properties based on a lead compound.

The term "chemical compound" is understood to mean a pure substance consisting of atoms of two or more chemical elements, where (in contrast to mixtures) the atomic species are in a fixed ratio to each other. A chemical compound has a defined chemical structure which reflects the structure at the molecular (or ionic) level. Preferably, the chemical compound is an organic compound. An "organic compound" is a chemical compound comprising carbon-hydrogen bonds (C-H bonds). Preferably, the chemical compound is an organic compound whose molecules are composed of only the following elements: Carbon (C), Hydrogen (H), Oxygen (O), Nitrogen (N), Sulfur (S), Fluorine (F), Chlorine (Cl), Bromine (Br), Iodine (I) and/or Phosphorus (P).

The term "lead compound" is understood to mean a chemical compound which serves as a starting point for chemical modifications in order to generate further chemical compounds with desired properties.

Typically, optimization of the lead structure is performed with respect to a plurality of properties that define a property profile. The properties may be physical properties, chemical properties, biological properties, and/or other properties.

Typical properties are biological activity, selectivity, toxicity, solubility, chemical stability and/or the like. Usually, each of the properties can be measured and specified by one or more values.

Given a lead compound and a target property profile, one or more chemical compounds are predicted by a machine learning model. A predicted chemical compounds is also referred to as "test compound" in this disclosure.

Such a "machine learning model", as used herein, may be understood as a computer implemented data processing architecture. The machine learning model can receive input data and provide output data based on that input data and on parameters of the machine learning model. The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training, where the loss function can quantify the deviations between the output and the target. The loss function may be chosen in such a way that it rewards a wanted relation between output and target and/or penalizes an unwanted relation between an output and a target. Such a relation can be, e.g., a similarity, or a dissimilarity, or another relation

The machine learning model of the present disclosure is now described in more detail, and it is described how the machine learning model is trained to perform the prediction tasks described herein. Afterwards it is described how the trained machine learning model can be used to predict chemical compounds.

The machine learning model of the present disclosure comprises an encoder-decoder structure, also referred to as autoencoder.

An autoencoder is usually used to learn efficient data encodings in an unsupervised manner. In general, the aim of an autoencoder is to learn a representation (encoding) for a set of data, typically for dimensionality reduction, by training the machine learning model to ignore "noise". Along with the reduction side (encoder), a reconstructing side (decoder) is learnt, where the autoencoder tries to generate from the reduced encoding a representation as close as possible to its original input.

A key feature of an autoencoder is an information bottleneck between the encoder and the decoder. This bottleneck, a continuous fixed-length vector, causes the machine learning model to learn a compressed representation that captures the most statistically salient information in the data.

A multitude of vectors representing a variety of different chemical compounds span a space, also known as latent space, latent feature space or embedding space. An important feature of latent space is that it is continuous. With the help of the encoder, a molecular representation of a chemical compound consisting of discrete elements is converted into a continuous vector in which the chemical compound is defined by numbers. The decoder can convert a continuous vector back into a discrete molecular representation

Autoencoders and their use to generate fixed-size representations of discrete molecular representations of chemical compounds in continuous latent space are well known and described in the prior art (see, e.g., R. Winter et al.: Learning continuous and data-driven molecular descriptors by translating equivalent chemical representations, Chem, Sci., 2019, 10, 1692-1701; R. Gomez-Bombarelli et al.: Automatic Chemical Design Using a Data-Driven Continuous Representation of Molecules, ACS Cent. Sci. 2018, 4, 268-276; S. Mohammadi et al.: Penalized Variational Autoencoderfor Molecular Design, DOI: 10.26434/chemrxiv.7977131.v2).

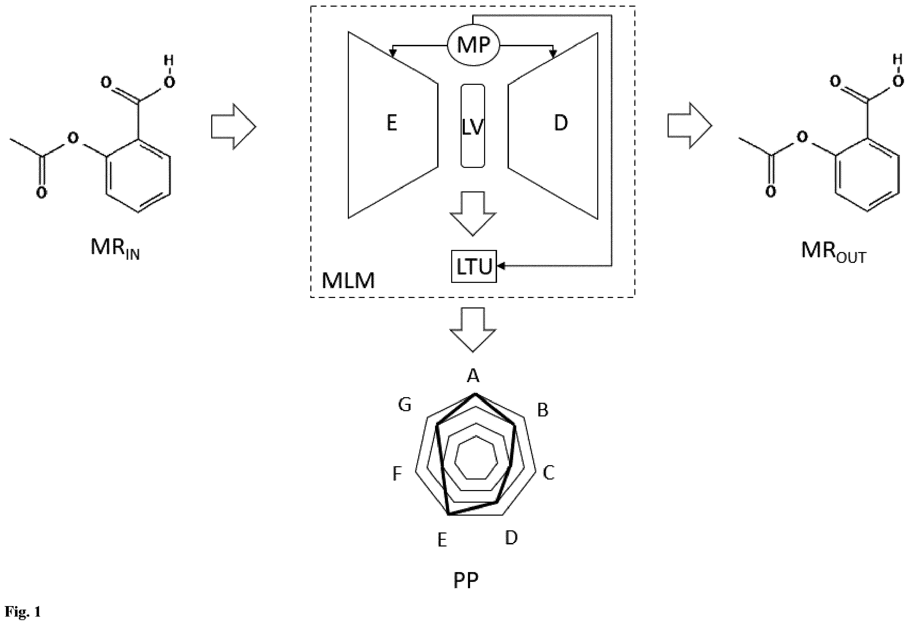

Fig. 1 shows schematically an example of a machine learning model of the present disclosure. The machine learning model MLM comprises an encoder E, a decoder D, and a linear transformation unit LTU.

The encoder E is configured to receive a molecular representation $MR_{IN}$ of a chemical compound and to generate, at least partially on the basis of the molecular representation $MR_{IN}$ and model parameters MP, a vector LV representing the chemical compound in continuous latent space.

The decoder D is configured to receive a vector LV representing a chemical compound in the continuous latent space and generate a molecular representation $MR_{OUT}$ at least partially on the basis of the vector LV and model parameters MP.

The linear transformation unit LTU is configured to receive a vector LV representing a chemical compound in the continuous latent space and predict a property profile $PP_{OUT}$ at least partially on the basis of the vector LV and model parameters MP.

Preferably, a variational autoencoder is used as autoencoder, as described for example in R. Gomez-Bombarelli et al.: Automatic Chemical Design Using a Data-Driven Continuous Representation of Molecules, ACS Cent. Sci. 2018, 4, 268-276; or S. Mohammadi et al.: Penalized Variational Autoencoder for Molecular Design, DOI: 10.26434/chemrxiv.7977131.v2.

The autoencoder can be trained in an unsupervised learning procedure. There are numerous databases that store molecular representations of chemical compounds that can be used to train the autoencoder, such as PubChem (https://pubchem.ncbi.nlmnihgov). Likewise, there are several publicly available databases in which properties of chemical compounds are stored such as PubChem and ZINC (http://zinc. docking. org).

The molecular representation of the chemical compound can, e.g., be a SMILES, InChI, CML or WIN representation. The simplified molecular-input line-entry system (SMILES) is a specification in the form of a line notation for describing the structure of chemical species using ASCII strings. The IUPAC International Chemical Identifier (InChI) is a textual identifier for chemical substances. Chemical Markup Language (CML) is an approach to managing molecular information using tools such as XML (Extended Markup Language) and Java. Wiswesser line notation (WLN) was one of the first line notations capable of precisely describing complex molecules.

The molecular representation of the chemical compound can also be a molecular graph. A molecular graph is a representation of the structural formula of a chemical compound in terms of graph theory. A molecular graph can be a labeled graph whose vertices correspond to the atoms of the compound and edges correspond to chemical bonds. Its vertices can be labeled with the kinds of the corresponding atoms and edges can be labeled with the types of bonds.

The molecular representation of the chemical compound can also be the IUPAC name of the chemical compound. In chemical nomenclature, the IUPAC nomenclature of organic chemistry is a method of naming organic chemical compounds as recommended by the International Union of Pure and Applied Chemistry (IUPAC). It is published in the Nomenclature of Organic Chemistry. Ideally, every possible organic compound should have a name from which an unambiguous structural formula can be created.

Further molecular representations of chemical compounds are possible.

In a preferred embodiment, the molecular representation is a canonical SMILES code. Typically, multiple equally valid SMILES codes can be generated for a molecule. Therefore, algorithms have been developed to generate the same SMILES string for a given molecule; of the many possible strings, these algorithms select only one (see, e.g., D. Weininger et al.: SMILES. 2nd algorithm for generation of unique SMILES notation, J Chem Inf Comp Sci 1989, 29(2):97e101). Canonical SMILES codes are unique for each structure.

The linear transformation unit serves as property prediction unit. It is configured to map a vector in the continuous latent space to a property vector representing a property profile. A linear transformation is a function from one vector space to another that respects the underlying (linear) structure of each vector space. In other words: a linear transformation is a mapping between two vector spaces that preserves the operations of vector addition and scalar multiplication.

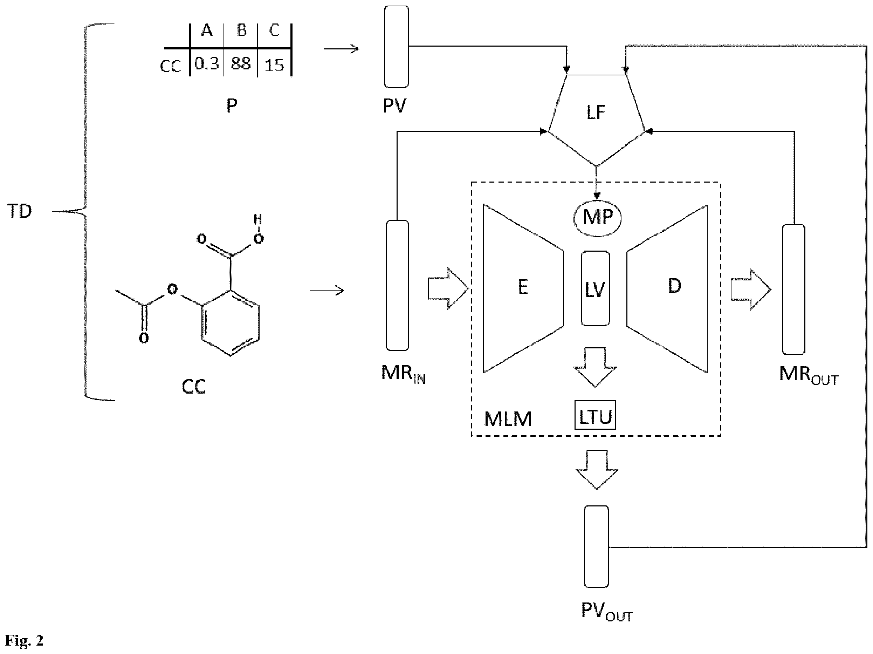

It is possible to train the machine learning model of the present disclosure to perform two tasks simultaneously: a reconstruction task and a property prediction task. Such a training is shown schematically in Fig. 2.

Fig. 2 shows the same machine learning model MLM as depicted in Fig. 1. The machine learning model MLM is trained using training data TD. The training data TD include, for each chemical compound CC of a plurality of chemical compounds, a molecular representation $MR_{IN}$ and at least one property P of the chemical compound CC. The term "plurality" as it is used herein means an integer greater than 1, usually greater than 10, preferably greater than 100.

In Fig. 2, only one training data set comprising a chemical compound CC and property data representing the at least one property P of the chemical compound CC are shown. The molecular representation $MR_{IN}$ of the chemical compound CC can be inputted by a user, read from a data storage, received from another computer system and/or generated from another representation of the chemical compound CC. The at least one property P is usually in the form of a numerical value. In the example shown in Fig. 2, three values are present for three parameters A, B and C. Each parameter A, B and C represents one or more properties of the chemical compound CC. Properties of chemical compounds can be determined empirically by measurements and/or retrieved from databases. An example of a publicly available database is the ZINC database (see, e.g., https://zinc.docking.org/).

A feature vector can be generated from the at least one property of the chemical compound. In machine learning, a feature vector is an *n*-dimensional vector of numerical features that represent an object (in this case one or more properties of a chemical compound), wherein *n* is an integer greater than 0. The term "feature vector" shall also include single values, matrices, tensors, and the like. Examples of feature vector generation methods can be found in various textbooks and scientific publications (see *e.g.* G.A Tsihrintzis, L.C. Jain: Machine Learning Paradigms: Advances in Deep Learning-based Technological Applications, in: Learning and Analytics in Intelligent Systems Vol. 18, Springer Nature, 2020, ISBN: 9783030497248; K. Grzegorczyk: Vector representations of text data in deep learning, Doctoral Dissertation, 2018, arXiv:1901.01695v1 [cs.CL]; M. Ilse et al.: Attention-based Deep Multiple Instance Learning, arXiv: 1802.04712v4 [cs.LG]).

In Fig. 2, the feature vector is shown as the property vector PV. For example, it is possible that each dimension of the feature vector (property vector) represents one of the parameters A, B, and C, and the vector elements represent the values for A, B, and C, respectively.

The molecular representation $MR_{IN}$ is fed to the encoder E as input data. The encoder E is configured and trained to generate, at least partially on the basis of the molecular representation $MR_{IN}$ and model parameters MP, a vector LV representing the chemical compound CC in continuous latent space. The decoder D is configured and trained to reconstruct, at least partially on the basis of the vector LV and model parameters MP, the molecular representation. In other words, the decoder D is configured and trained to generate and output, at least partially on the basis of the vector LV and model parameters MP, a molecular representation $MR_{OUT}$ of the chemical compound which comes as close as possible to the molecular representation $MR_{IN}$. Simultaneously, the linear transformation unit LTU is configured and trained to predict, at least partially on the basis of the vector LV and model parameters MP, the at least one property P. In other words, the linear transformation unit LTU is configured to generate and output a property vector $PV_{OUT}$ which comes as close as possible to the property vector PV.

The deviations between i) the target molecular representation $MR_{IN}$ and the outputted molecular representation $MR_{OUT}$, and ii) the target property vector PV and the outputted property vector $PV_{OUT}$ can be quantified using a loss function LF. Typically, the loss function LF comprises two terms, a first term that quantifies the deviations between the target molecular representation $MR_{IN}$ and the outputted molecular representation $MR_{OUT}$, and a second term that quantifies the deviations between the target property vector PV and the outputted property vector $PV_{OUT}$. In the loss function, the two terms can be added. In the loss function, the two terms may have different weights. The weights may also vary during training.

An examples of loss functions for the reconstruction task (first term) is cross-entropy loss. Examples of a loss function for the prediction task (second term) are: L1 loss and/or mean squared error.

It is possible to first train the autoencoder (reconstruction task) alone and then train the linear transformation unit (prediction task) or then train the linear transformation unit together with the autoencoder (combined reconstruction and prediction task).

The loss values calculated using the loss function can be used to modify model parameters to increase the accuracy with which the machine learning model reconstructs the molecular representation and/or predicts the at least one property. For example, a high loss value may mean that one or more model parameters need to be modified to a high degree.

Usually, an optimization procedure such as a gradient descent procedure is used to modify the model parameters in a way that leads to a reduction of loss values.

The machine learning model can be trained based on the training data until a predefined accuracy has been achieved (until the loss values have reached a pre-defined minimum).

A cross-validation method can be employed to split the training data into a training dataset and a validation dataset. The training dataset can be used in the training of the machine learning model. The validation dataset can be used to verify that the results of the trained machine learning are generalizable.

Once the machine learning model is trained, it can be used for prediction purposes.

According to the present disclosure, the trained machine learning model is used to propose (predict) one or more test compounds with a desired property profile based on a lead compound.

So, a lead compound is identified, and a target property profile is defined.

Lead compound and target property profile can be specified by a user. Lead compound and target property profile can be entered into the computer system of the present disclosure by the user or stored in a data memory and read out by the computer system of the present disclosure.

If a molecular representation of the lead compound is not yet available, one can be generated from another representation according to the usual procedures described in the prior art.

Analogously, a target property vector representing the target property profile can be generated from a target property profile that is, for example, available in the form of a table.

The linear transformation unit of the trained machine learning model is used to map the target property vector representing the target property profile to the continuous latent space.

As described above, the linear transformation unit represents a linear function that maps a vector in continuous latent space to a property vector. An inverse function can be determined for the linear function, and this inverse function can be used to map the target property vector to the continuous latent space.

Since there are usually many chemical compounds that satisfy the target property profile, a subset of latent space is determined by the mapping procedure. All points that lie in this subset are representations of potential chemical compounds that satisfy the target property profile.

In a next step, one or more representations of those chemical compounds that show structural similarity to the lead compound are identified in the subset.

This is achieved by generating the vector in the continuous latent space of the lead compound by means of the encoder and projecting the vector onto the subset. In other words, the molecular representation representing the lead compound is inputted into the encoder of the trained machine learning model, and the encoder generates a vector representing the lead compound in the continuous latent space. Then, the vector representing the lead compound in the continuous latent space is projected onto the subset representing the target property profile in the continuous latent space.

The result of the projection is a first vector representing a first test compound in the continuous latent space.

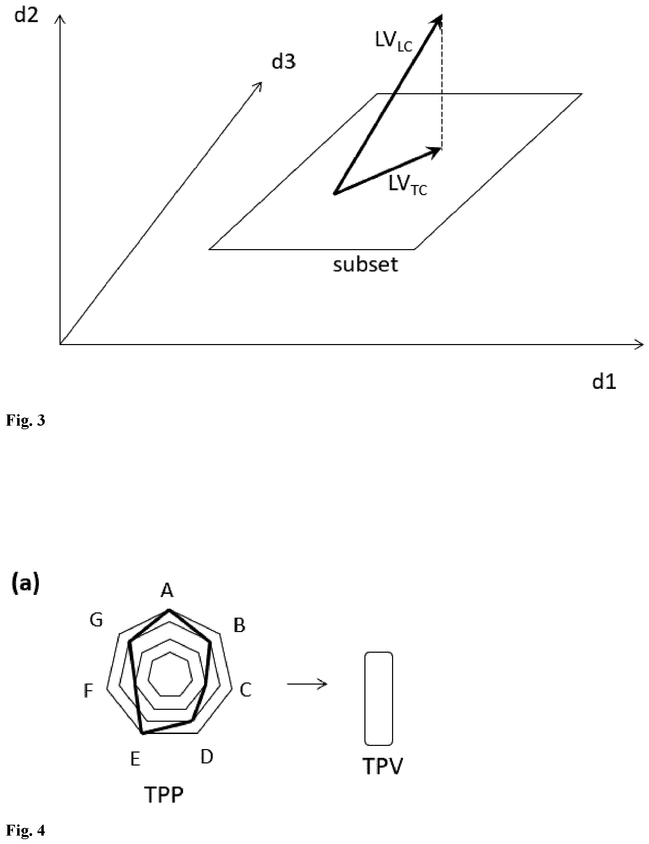

The projection of the vector representing the lead compound onto the subset of the continuous latent space representing the target property profile is schematically shown in Fig. 3.

In the example shown in Fig. 3, the continuous latent space comprises only three dimensions. Usually, it contains a lot more dimension, such as 100 or 512, or another number. The three dimensions form a Cartesian coordinate system with the axes d1, d2 and d3. The plane shown in the coordinate system is the subset of the continuous latent space representing the target profile.

A first vector $LV_{LC}$ represents the lead compound in the continuous latent space. This vector $LV_{LC}$ is projected onto the plane (subset). The result of the projection is the vector $LV_{TC}$. The vector $LV_{TC}$ represents a first test compound in the continuous latent space.

In a next step, the molecular structure of the first test compound can be determined. This can be done by inputting the vector representing the first test compound in the continuous latent space into the decoder of the trained machine learning model. The decoder outputs the molecular representation of the first test compound.

In a next step, it can be checked whether the molecular representation is a valid molecular representation, i. e., a representation of a chemical structure of a chemical compound that can actually exist, i. e., can be synthesized.

If the molecular representation is a SMILES code, this SMILES code can be validated, for example, using the freely available open-source cheminformatics software RDKit (see, e.g., http://www.rdkit.org).

Invalid molecular representations may be discarded.

In a next step, it is checked whether the property profile of the first test compound matches the target property profile. The (optionally validated) molecular representation of the first test compound is inputted into the encoder of the trained machine learning model. The encoder generates a vector representing the first test compound in the continuous latent space. The vector representing the first test compound in the continuous latent space is then inputted into the linear transformation unit in order to predict the property profile of the first test compound. The linear transformation unit outputs a property vector representing the property profile of the first test compound. The property vector of the first test compound can then be compared with the target property vector representing the target property profile.

In such a comparison, a measure of similarity of the two vectors can be determined. A measure of the similarity of two vectors can be, for example, a similarity value, such as the cosine similarity, or a distance measure, such as the Euclidean distance, the Manhattan distance, Chebyshev distance, Minkowski distance, weighted Minkowski distance, Mahalanobis distance, Hamming distance, Canberra distance, Bray Curtis distance, or a combination thereof.

A distance $d(TPV, PV_{TC})$ between a target propety vector TPV and a property vector $PV_{TC}$ representing the properties of a test compound can be converted into a similarity value $s(TPV, PV_{TC})$, e.g., by the following

equation:

$$s(\text{TPV}, \text{PV}_{\text{TC}}) = \frac{1}{1 + d(\text{TPV}, \text{PV}_{\text{TC}})}$$

The similarity value (or the distance value or any other measure of the similarity of the two vectors TPV and PV$_{\text{TC}}$) can be compared with a pre-defined threshold. This is explained by the example of a similarity value which is always positive and takes the value 1 (or 100%) if two vectors are identical and takes the value 0 if two vectors have no similarity. For example, the pre-defined threshold may be 0.8 or 0.85 or 0.9 or 0.91 or 0.95 or 0.99 or some other value.

If the similarity value is smaller than the pre-defined threshold, it may mean that the property profile of the first test compound is so far away from the target profile that the test compound is not a promising candidate for further investigation and can be discarded.

If the similarity value is equal to or greater than the pre-defined threshold, it may mean that the property profile of the first test compound is so close to the target profile that the test compound should be investigated further. The molecular representation of the first test compound can be outputted. For example, the molecular representation of the first test compound can be displayed on a monitor, printed on a printer, stored on a data storage, or transmitted to a separate computer system.

If the property profile of the first test compound has a predefined similarity to the target property profile, e.g., if a similarity value between the two vectors is equal to or greater than a pre-defined threshold, measures for synthesis and/or characterization (testing of properties) of the first test compound can also be initiated.

"Initiating synthesis and/or characterization" may mean: identifying chemical compounds for synthesis of the first test compound, ordering chemical compounds for synthesis of the first test compound, reserving laboratory space and/or equipment for synthesis of the first test compound, ordering laboratory personnel for synthesis of the first test compound, ordering characterization of the first test compound (e. g., to confirm the predicted property profile of the first test compound), reserving laboratory space and/or equipment for characterization of the first test compound, ordering equipment for characterization of the test compound. Said actions may be performed by the computer system of the present disclosure, for example, by transmitting a corresponding message to one or more persons, creating calendar entries, initiating orders, and/or the like. It is also possible that the computer system of the present disclosure is in communication with a synthesis robot and can cause the synthesis robot to synthesize the first test compound. It is also possible that the computer system of the present disclosure is in communication with a device for characterizing the first test compound and can cause the device to perform a characterization of the first test compound (e.g., in an assay to determine biological activity of the first test compound).

In the event that the first test compound is not a valid chemical compound and/or the properties of the first test compound do not match the desired properties (the target property profile), a second test compound can be predicted by the machine learning model. There are several possibilities for the prediction of a second test compound. Three examples are given below.

For example, a new lead compound can be selected. A molecular representation of the new lead compound can be inputted into the encoder of the trained machine learning model in order to generate a vector representing the new lead compound in the continuous latent space. The vector can then be projected onto the subset representing the target property profile in the continuous latent space. The result of the projection is a vector representing a second test compound in the continuous latent space. The second test compound can then be treated in the same way as the first test compound and as described herein.

For example, the target property profile can be modified. Often, it is not necessary for a test compound to have a specific value of a parameter; it is sufficient if the value of the parameter is within a predefined range. Thus, it is easy to change one or more values of parameters defining one or more target properties without deteriorating the quality of test compounds predicted on the basis of the changed target property profile. A feature vector (target property vector) can then be generated from the modified target property profile. The feature vector (target property vector) can be mapped to the continuous latent space via the linear transformation unit. The result is a new subset of the latent space that represents the modified target property profile in the latent space. The vector representing the lead compound can be projected to the new subset. The result of the projection is a vector representing a second test compound in the continuous latent space. The second test compound can then be treated in the same way as the first test compound and as described herein.

For example, on the basis of the vector representing the first test compound in the continuous latent space (the first vector), a vector representing a second test compound can be generated (the second vector) by moving away from the endpoint of the first vector by a pre-defined distance in a pre-defined direction within the continuous latent space. The point one will get to, is the endpoint of the second vector (the other point of the second vector is the origin). When starting from the endpoint of the first vector, one can move within the subset or outside the subset representing the target property profile. The further one moves away from the first vector, the more one or more properties of the second test compound may change and/or the more the chemical structure of the second test compound may change (compared to the first test compound), depending on which property/properties and/or structural feature(s) is/are represented by the direction in which one moves.

Combinations of the examples given above are also possible.

A preferred embodiment of the method according to the present disclosure is shown schematically in Fig. 4 (a) to Fig. 4 (i).

Fig. 4 (a) shows the step of generating a target property vector TPV representing a target property profile TPP from the target property profile TPP.

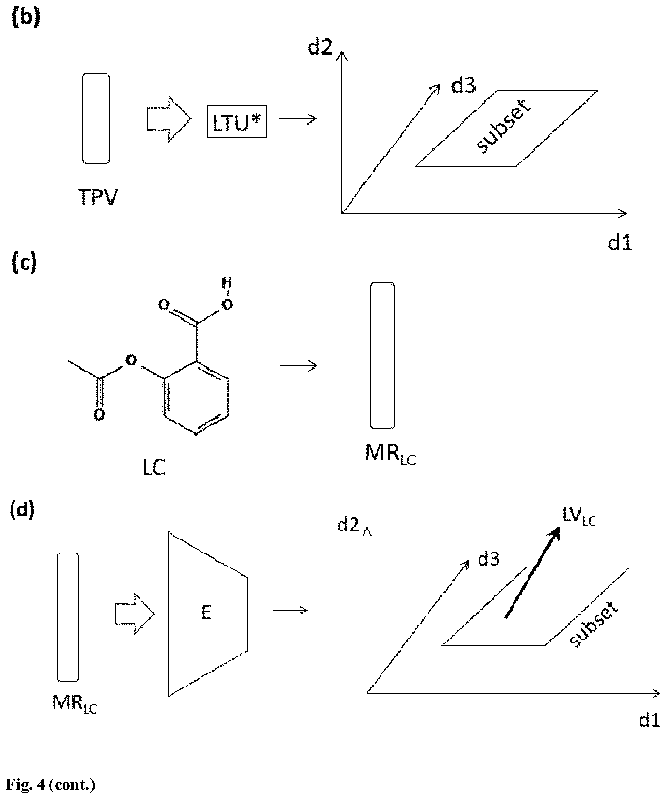

Fig. 4 (b) shows the step of determining a subset of the continuous latent space representing the target property profile in the continuous latent space from the target property vector TPV. The subset is determined by mapping the target property vector TPV to the continuous latent space via the linear transformation unit LTU*. The asterisk * indicates that the linear function represented by the linear transformation unit LTU has been replaced by its inverse function.

Fig. 4 (c) shows the step of generating a molecular representation $MR_{LC}$ from the lead compound LC.

Fig. 4 (d) shows the step of generating a vector $LV_{LC}$ representing the lead compound in the continuous latent space from the molecular representation $MR_{LC}$. The molecular representation $MR_{LC}$ is inputted into the encoder E of the trained machine learning model and the encoder outputs the vector $LV_{LC}$.

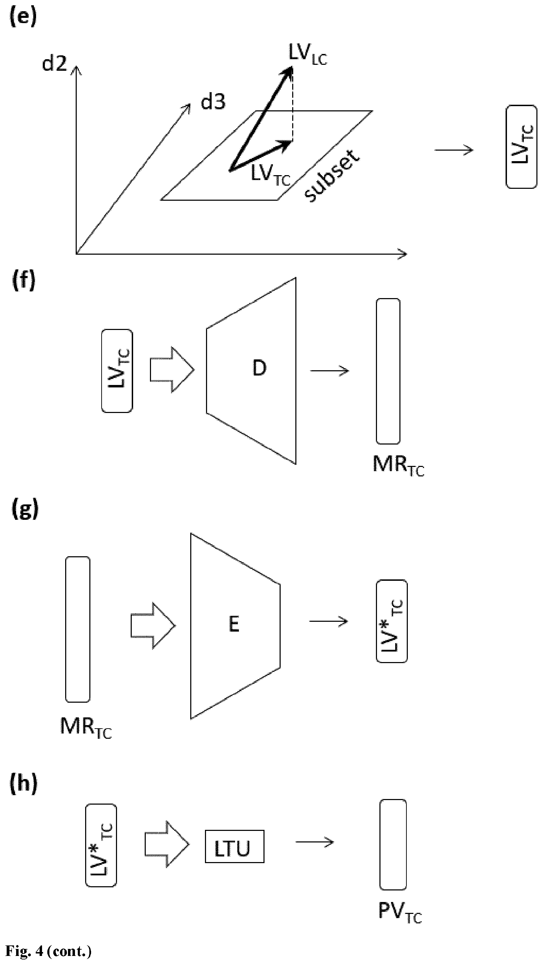

Fig. 4 (e) shows the step of projecting the vector $LV_{LC}$ onto the subset representing the target property profile in the continuous latent space. The result of the projection is a vector $LV_{TC}$ representing a first test compound in the continuous latent space.

Fig. 4 (f) shows the step of generating a molecular representation $MR_{TC}$ of the first test compound from the vector $LV_{TC}$. The vector $LV_{TC}$ is inputted into the decoder D of the trained machine learning model and the decoder D outputs the molecular representation $MR_{TC}$.

Fig. 4 (g) shows the step of generating a vector $LV^*_{TC}$ representing the first test compound in the continuous latent space from the molecular representation $MR_{TC}$ of the first test compound. The molecular representation $MR_{TC}$ is inputted into the encoder E of the trained machine learning model and the encoder E outputs the vector $LV^*_{TC}$. The asterisk * serves to distinguish the vector $LV^*_{TC}$ in Fig. 4 (g) and Fig. 4 (h) from the vector $LV_{TC}$ in Fig. 4 (e) and (f). Both vectors, $LV_{TC}$ and $LV^*_{TC}$, represent the first test compound in the continuous latent space. One might assume that the vectors should be the same. However, due to the probabilistic nature of the machine learning model, the vectors usually differ. $LV^*_{TC}$ refers to the vector generated from the molecular representation $MR_{TC}$ of the first test compound using the encoder E of the trained machine learning model; $LV_{TC}$ refers to the vector generated by projecting the vector $LV_{LC}$ of the lead compound onto the subset representing the target property profile in the continuous latent space.

Fig. 4 (h) shows the step of generating a property vector $PV_{TC}$ representing the properties of the first test compound from the vector $LV^*_{TC}$ representing the first test compound in the continuous latent space. The vector $LV^*_{TC}$ is inputted into the linear transformation unit LTU of the trained machine learning model and the linear transformation unit LTU outputs the property vector $PV_{TC}$.

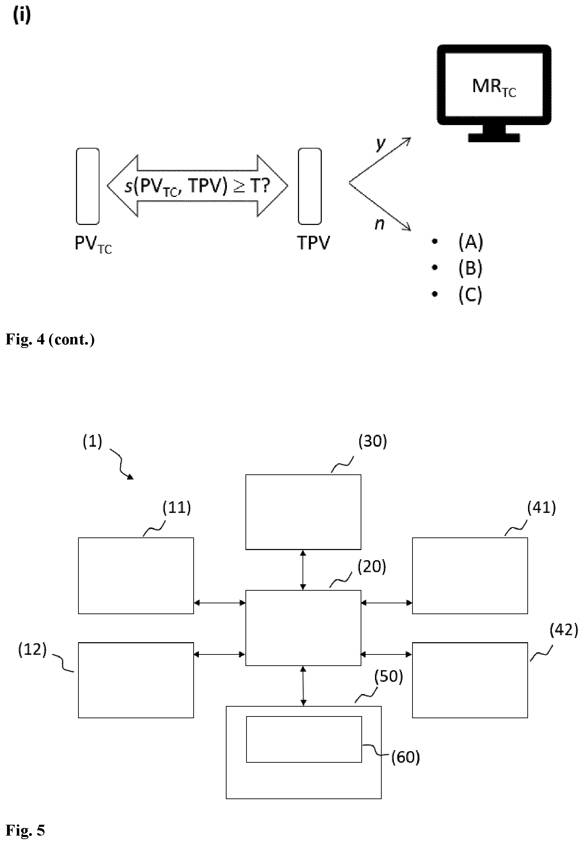

Fig. 4 (i) shows the step of comparing the properties of the first test compound represented by the property vector $PV_{TC}$ with the target property profile represented by the target property vector TPV ("$s(PV_{TC}, TPV) \geq T?$"). In the example shown in Fig. 4 (i), a similarity values is calculated, the similarity value s quantifying the similarity between the vector $PV_{TC}$ and the vector TPV.

If the similarity value s is equal to or greater than a pre-defined threshold T ("*y*"), the first test compound is selected for further investigation; the molecular representation $MR_{TC}$ of the first test compound and/or any other representation of the first test compound can be outputted, e.g., on a monitor. If the similarity value *s* is smaller than the pre-defined threshold T ("*n*"), a second test compound is identified by (A) changing the lead compound LC and/or (B) by modifying the target property profile PP and/or (C) by moving away from the vector $LV_{TC}$ in the continuous latent space.

The approach described herein effectively and efficiently leads to new candidates for lead structure optimization, wherein the new candidates (predicted test compounds) represent valid chemical compounds and exhibit properties that match the target property profile.

The operations in accordance with the teachings herein may be performed by at least one computer system specially constructed for the desired purposes or general-purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application The term "computer" / "computer system" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e. g., within registers and/or memories of at least one computer or processor. The term processor includes a single processing unit or a plurality of distributed or remote such units.

Fig. 5 illustrates a computer system (1) according to some example implementations of the present disclosure in more detail. The computer system (1) may include one or more of each of a number of components such as, for example, processing unit (20) connected to a memory (50) (e.g., storage device).

The processing unit (20) may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory (50) of the same or another computer.

The processing unit (20) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (50) is generally any piece of computer hardware that is capable of storing information such as, for example, data, computer programs (e. g., computer-readable program code (60)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), lightemitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

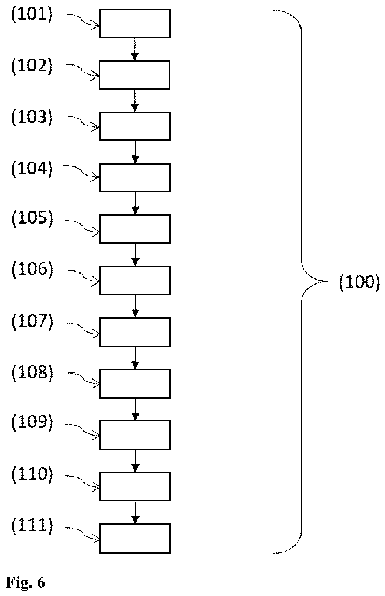

Fig. 6 shows a preferred embodiment of the computer-implemented method of predicting a first test compound. The method (100) comprises the steps:

(101) providing a trained machine learning model, the trained machine learning model comprising an encoder, a decoder, and a linear transformation unit,

- wherein the encoder is configured to convert a discrete molecular representation of a chemical compound into a vector in continuous latent space,

- wherein the decoder is configured to convert a vector in the continuous latent space into a discrete molecular representation of a chemical compound,

- wherein the linear transformation unit is configured to map a vector in the continuous latent space to a property vector representing a property profile,

(102) receiving a target property vector representing a target property profile,

(103) mapping the target property vector to the continuous latent space via the linear transformation unit, thereby determining a subset in the continuous latent space,

(104) receiving a molecular representation of a lead compound,

(105) converting the molecular representation of the lead compound to a vector representing the lead compound in the continuous latent space via the encoder,

(106) projecting the vector representing the lead compound in the continuous latent space onto the subset, thereby receiving a first vector representing the first test compound in the continuous latent space,

(107) generating a discrete molecular representation of the first test compound using the decoder,

(108) inputting the discrete molecular representation of the first test compound into the encoder, thereby generating a second vector representing the first test compound in the continuous latent space,

(109) inputting the second vector representing the first test compound in the continuous latent space into the linear transformation unit, thereby generating a property vector representing a property profile of the first test compound,

(110) comparing the property profile of the first test compound with the target property profile,

(111) in case the property profile of the first test compound has a pre-defined similarity to the target property profile: outputting the discrete molecular representation of the first test compound and/or another representation of the first test compound.

## Claims

1. A computer-implemented method, the method comprising:

- providing a trained machine learning model (MLM), the trained machine learning model (MLM) comprising an encoder (E), a decoder

(D), and a linear transformation unit (LTU, LTU*),

• wherein the encoder (E) is configured and trained to convert a discrete molecular representation ($MR_{IN}$) of a chemical compound (CC) into a vector (LV) in continuous latent space,
• wherein the decoder (D) is configured and trained to convert a vector (LV) in the continuous latent space into a discrete molecular representation ($MR_{OUT}$) of a chemical compound (CC),
• wherein the linear transformation unit (LTU, LTU*) is configured and trained to map a vector (LV) in the continuous latent space to a property vector ($PV_{OUT}$) representing a property profile,

- receiving a target property vector (TPV) representing a target property profile (TPP),
- mapping the target property vector (TPV) to the continuous latent space via the linear transformation unit (LTU*), thereby determining a subset in the continuous latent space,
- receiving a molecular representation ($MR_{LC}$) of a lead compound (LC),
- converting the molecular representation ($MR_{LC}$) of the lead compound (LC) to a vector ($LV_{LC}$) representing the lead compound (LC) in the continuous latent space via the encoder (E),
- projecting the vector ($LV_{LC}$) representing the lead compound (LC) in the continuous latent space onto the subset, thereby generating a first vector ($LV_{TC}$) representing a first test compound in the continuous latent space,
- inputting the first vector ($LV_{TC}$) representing the first test compound in the continuous latent space into the decoder (D), thereby generating a discrete molecular representation ($MR_{TC}$) of the first test compound,
- inputting the discrete molecular representation ($MR_{TC}$) of the first test compound into the encoder (E), thereby generating a second vector ($LV^*_{TC}$) representing the first test compound in the continuous latent space,
- inputting the second vector ($LV^*_{TC}$) representing the first test compound in the continuous latent space into the linear transformation unit (LTU), thereby generating a property vector ($PV_{TC}$) representing a property profile of the first test compound,
- comparing the property profile of the first test compound with the target property profile (TPP),
- in case the property profile of the first test compound has a pre-defined similarity to the target property profile (TPP): outputting the discrete molecular representation ($MR_{TC}$) of the first test compound and/or another representation of the first test compound.

2. The method according to claim 1, wherein the target property profile (TPP) comprises one or more target values of one of more of the following properties: biological activity, selectivity, toxicity, solubility, chemical stability.

3. The method according to claim 1 or 2, wherein the encoder (E) and the decoder (D) are parts of a variational autoencoder.

4. The method according to any one of claims 1 to 3, wherein the molecular representation ($MR_{LC}$) of the lead compound and the molecular representation ($MR_{TC}$) of the first test compound are SMILES codes, preferably canonical SMILES codes.

5. The method according to any one of claims 1 to 4, wherein the trained machine learning model (MLM) was trained on training data (TD), the training data (TD) comprising, for each chemical compound (CC) of a plurality of chemical compounds, a molecular representation ($MR_{IN}$) of the chemical compound (CC) and at least one property (P) representing a property profile of the chemical compound (CC).

6. The method according claim 5, wherein training of the machine learning model (MLM) comprises:

- for each chemical compound (CC) of the plurality of chemical compounds:

◦ inputting the molecular representation ($MR_{IN}$) of the chemical compound (CC) into the encoder (E),
◦ receiving from the decoder (D) an output molecular representation ($MR_{OUT}$),
◦ quantifying the differences between the inputted molecular representation ($MR_{IN}$) and the output molecular representation ($MR_{OUT}$) using a first loss term,
◦ receiving from the linear transformation unit (LTU) a predicted property profile ($PV_{OUT}$),
◦ quantifying the differences between the property profile and the predicted property profile ($PV_{OUT}$) using a second loss term,
◦ computing a loss using a loss function (LF), the loss function (LF) comprising the first loss term and the second loss term,
◦ modifying parameters of the machine learning model (MLM) based on the computed loss.

7. The method according to any one of claims 1 to 6, the property profile of the first test compound and the target property profile (TPP) are represented by feature vectors (PV$_{TC}$, TPV).

8. The method according claim 7, wherein the step comparing the property profile of the first test compound with the target property profile (TPP), comprises:

- computing a similarity value (*s*), the similarity value (*s*) quantifying the similarity between the feature vector (PV$_{TC}$) representing the property profile of the first test compound and the feature vector (TPV) representing the target property profile (TPP),
- comparing the similarity value with a pre-defined threshold (T).

9. The method according to any one of claims 1 to 8, further comprising the steps:

- modifying the target property profile (TPP),
- mapping the modified target property vector to the continuous latent space via the linear transformation unit (LTU*), thereby determining a modified subset in the continuous latent space,
- projecting the vector (LV$_{LC}$) representing the lead compound (LC) in the continuous latent space onto the modified subset, thereby receiving a second vector representing a second test compound in the continuous latent space,
- generating a discrete molecular representation of the second test compound using the decoder,
- inputting the discrete molecular representation of the second test compound into the encoder and determining a property profile for the second test compound via the linear transformation unit (LTU),
- comparing the property profile of the second test compound with the target property profile,
- in case the property profile of the second test compound has a pre-defined similarity to the target property profile: outputting the discrete molecular representation of the second test compound and/or another representation of the second test compound.

10. The method according to any one of claims 1 to 9, further comprising the steps:

- moving a pre-defined distance in a pre-defined direction from a point representing the first test compound in the continuous latent, thereby defining a second vector representing a second test compound in the continuous latent space,
- generating a discrete molecular representation of the second test compound using the decoder (D),
- inputting the discrete molecular representation of the second test compound into the encoder (E) and determining a property profile for the second test compound via the linear transformation unit (LTU),
- comparing the property profile of the second test compound with the target property profile (TPP),
- in case the property profile of the second test compound has a pre-defined similarity to the target property profile (TPP): outputting the discrete molecular representation of the second test compound and/or another representation of the second test compound.

11. The method according to any one of claims 1 to 10, further comprising:

- initiating synthesis and/or characterization of the first test compound.

12. A computer system comprising:

a processing unit (20); and
a memory (50) storing an application program configured to perform, when executed by the processing unit (20), an operation, the operation comprising:

- providing a trained machine learning model (MLM), the trained machine learning model (MLM) comprising an encoder (E), a decoder (D), and a linear transformation unit (LTU, LTU*),

• wherein the encoder (E) is configured and trained to convert a discrete molecular representation (MR$_{IN}$) of a chemical compound (CC) into a vector (LV) in continuous latent space,
• wherein the decoder (D) is configured and trained to convert a vector (LV) in the continuous latent space into a discrete molecular representation (MR$_{OUT}$) of a chemical compound (CC),
• wherein the linear transformation unit (LTU, LTU*) is configured and trained to map a vector (LV) in the continuous latent space to a property vector (PV$_{OUT}$) representing a property profile,

- receiving a target property vector (TPV) representing a target property profile (TPP),
- mapping the target property vector (TPV) to the continuous latent space via the linear transformation unit (LTU*), thereby determining a subset in the continuous latent space,
- receiving a molecular representation ($MR_{LC}$) of a lead compound (LC),
- converting the molecular representation ($MR_{LC}$) of the lead compound (LC) to a vector ($LV_{LC}$) representing the lead compound (LC) in the continuous latent space via the encoder (E),
- projecting the vector ($LV_{LC}$) representing the lead compound (LC) in the continuous latent space onto the subset, thereby generating a first vector ($LV_{TC}$) representing a first test compound in the continuous latent space,
- inputting the first vector ($LV_{TC}$) representing the first test compound in the continuous latent space into the decoder (D), thereby generating a discrete molecular representation ($MR_{TC}$) of the first test compound,
- inputting the discrete molecular representation ($MR_{TC}$) of the first test compound into the encoder (E), thereby generating a second vector ($LV^*_{TC}$) representing the first test compound in the continuous latent space,
- inputting the second vector ($LV^*_{TC}$) representing the first test compound in the continuous latent space into the linear transformation unit (LTU), thereby generating a property vector ($PV_{TC}$) representing a property profile of the first test compound,
- comparing the property profile of the first test compound with the target property profile (TPP),
- in case the property profile of the first test compound has a pre-defined similarity to the target property profile (TPP): outputting the discrete molecular representation ($MR_{TC}$) of the first test compound and/or another representation of the first test compound.

13. A non-transitory computer readable medium having stored thereon software instructions that, when executed by a processing unit (20) of a computer system (1), cause the computer system (1) to execute the following steps:

- providing a trained machine learning model (MLM), the trained machine learning model (MLM) comprising an encoder (E), a decoder (D), and a linear transformation unit (LTU, LTU*),

• wherein the encoder (E) is configured and trained to convert a discrete molecular representation ($MR_{IN}$) of a chemical compound (CC) into a vector (LV) in continuous latent space,

• wherein the decoder (D) is configured and trained to convert a vector (LV) in the continuous latent space into a discrete molecular representation ($MR_{OUT}$) of a chemical compound (CC),
• wherein the linear transformation unit (LTU, LTU*) is configured and trained to map a vector (LV) in the continuous latent space to a property vector ($PV_{OUT}$) representing a property profile,

- receiving a target property vector (TPV) representing a target property profile (TPP),
- mapping the target property vector (TPV) to the continuous latent space via the linear transformation unit (LTU*), thereby determining a subset in the continuous latent space,
- receiving a molecular representation ($MR_{LC}$) of a lead compound (LC),
- converting the molecular representation ($MR_{LC}$) of the lead compound (LC) to a vector ($LV_{LC}$) representing the lead compound (LC) in the continuous latent space via the encoder (E),
- projecting the vector ($LV_{LC}$) representing the lead compound (LC) in the continuous latent space onto the subset, thereby generating a first vector ($LV_{TC}$) representing a first test compound in the continuous latent space,
- inputting the first vector ($LV_{TC}$) representing the first test compound in the continuous latent space into the decoder (D), thereby generating a discrete molecular representation ($MR_{TC}$) of the first test compound,
- inputting the discrete molecular representation ($MR_{TC}$) of the first test compound into the encoder (E), thereby generating a second vector ($LV^*_{TC}$) representing the first test compound in the continuous latent space,
- inputting the second vector ($LV^*_{TC}$) representing the first test compound in the continuous latent space into the linear transformation unit (LTU), thereby generating a property vector ($PV_{TC}$) representing a property profile of the first test compound,
- comparing the property profile of the first test compound with the target property profile (TPP),
- in case the property profile of the first test compound has a pre-defined similarity to the target property profile (TPP): outputting the discrete molecular representation ($MR_{TC}$) of the first test compound and/or another representation of the first test compound.

Fig. 1

| | A | B | C |
|---|---|---|---|
| CC | 0.3 | 88 | 15 |

P

CC

TD

PV

LF

MR$_{IN}$

MR$_{OUT}$

MP

LV

D

E

LTU

MLM

PV$_{OUT}$

Fig. 2

d2

d3

LV$_{LC}$

LV$_{TC}$

subset

d1

**Fig. 3**

(a)

A

G

B

F

C

E

D

TPP

TPV

**Fig. 4**

**(b)**

**(c)**

**(d)**

**Fig. 4 (cont.)**

**(e)**

**(f)**

**(g)**

**(h)**

**Fig. 4 (cont.)**

**(i)**

$s(\text{PV}_{\text{TC}}, \text{TPV}) \geq \text{T}?$

$\text{PV}_{\text{TC}}$     TPV

$\text{MR}_{\text{TC}}$

$y$

$n$

- (A)
- (B)
- (C)

**Fig. 4 (cont.)**

(1)    (30)    (41)

(11)    (20)

(12)    (42)

(50)

(60)

**Fig. 5**

Fig. 6

Europäisches
Patentamt

European
Patent Office

Office européen
des brevets

# EUROPEAN SEARCH REPORT

**Application Number**

**EP 23 16 6704**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | Mohammadi Sadegh ET AL: "Penalized Variational Autoencoder for Molecular Design", ChemRxiv, 12 April 2019 (2019-04-12), XP055961579, DOI: 10.26434/chemrxiv.7977131.v2 Retrieved from the Internet: URL:https://chemrxiv.org/engage/chemrxiv/article-details/60c74169f96a0012ee286438 [retrieved on 2022-09-15] * the whole document * | 1-13 | INV. G16C20/50 G06N3/00 G16C20/70 |
| X | WO 2021/165887 A1 (INSILICO MEDICINE IP LTD [CN]) 26 August 2021 (2021-08-26) * the whole document * | 1-13 | |
| X | WEI RUOQI ET AL: "Recent Advances in Variational Autoencoders With Representation Learning for Biomedical Informatics: A Survey", IEEE ACCESS, IEEE, USA, vol. 9, 31 December 2020 (2020-12-31), pages 4939-4956, XP011835793, DOI: 10.1109/ACCESS.2020.3048309 * Section III. A Molecular desing; page 4943 - page 4945 * | 1-13 | TECHNICAL FIELDS SEARCHED (IPC) G16C G06N |
| X | DANIEL C ELTON ET AL: "Deep learning for molecular generation and optimization - a review of the state of the art", ARXIV.ORG, CORNELL UNIVERSITY LIBRARY, 201 OLIN LIBRARY CORNELL UNIVERSITY ITHACA, NY 14853, 11 March 2019 (2019-03-11), XP081270483, * the whole document * | 1-13 | |

-/--

The present search report has been drawn up for all claims

| Place of search | Date of completion of the search | Examiner |
|---|---|---|
| The Hague | 21 August 2023 | Denoual, Matthieu |

EPO FORM 1503 03.82 (P04C01)

**page 1 of 2**

Europäisches
Patentamt

European
Patent Office

Office européen
des brevets

**EUROPEAN SEARCH REPORT**

Application Number

EP 23 16 6704

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BENJAMIN SANCHEZ-LENGELING ET AL: "Inverse molecular design using machine learning: Generative models for matter engineering", SCIENCE, vol. 361, no. 6400, 27 July 2018 (2018-07-27), pages 360-365, XP055634377, US ISSN: 0036-8075, DOI: 10.1126/science.aat2663 * the whole document * ----- | 1-13 | |
| | | | TECHNICAL FIELDS SEARCHED (IPC) |

The present search report has been drawn up for all claims

| Place of search | Date of completion of the search | Examiner |
|---|---|---|
| The Hague | 21 August 2023 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 6704

This annex lists the patent family members relating to the patent documents cited in the above-mentioned European search report.
The members are as contained in the European Patent Office EDP file on
The European Patent Office is in no way liable for these particulars which are merely given for the purpose of information.

21-08-2023

| Patent document cited in search report | | Publication date | Patent family member(s) | | Publication date |
|---|---|---|---|---|---|
| WO 2021165887 | A1 | 26-08-2021 | CN 115104105 | A | 23-09-2022 |
| | | | EP 4107668 | A1 | 28-12-2022 |
| | | | US 2023075100 | A1 | 09-03-2023 |
| | | | WO 2021165887 | A1 | 26-08-2021 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Automatic Chemical Design Using a Data-Driven Continuous Representation of Molecules. *ACS Cent. Sci.,* 2018, vol. 4, 268-276
- *Penalized Variational Autoencoder for Molecular Design*
- **R. WINTER et al.** Learning continuous and data-driven molecular descriptors by translating equivalent chemical representations. *Chem, Sci.,* 2019, vol. 10, 1692-1701
- **R. GOMEZ-BOMBARELLI et al.** Automatic Chemical Design Using a Data-Driven Continuous Representation of Molecules. *ACS Cent. Sci.,* 2018, vol. 4, 268-276
- **S. MOHAMMADI et al.** *Penalized Variational Autoencoderfor Molecular Design*

- **S. MOHAMMADI et al.** *Penalized Variational Autoencoder for Molecular Design*
- **D. WEININGER et al.** SMILES. 2nd algorithm for generation of unique SMILES notation. *J Chem Inf Comp Sci,* 1989, vol. 29 (2), 97e101
- Jain: Machine Learning Paradigms: Advances in Deep Learning-based Technological Applications. **G.A TSIHRINTZIS, L.C.** Learning and Analytics in Intelligent Systems. Springer Nature, 2020, vol. 18
- **K. GRZEGORCZYK.** Vector representations of text data in deep learning, Doctoral Dissertation. *arXiv:1901.01695v1,* 2018
- **M. ILSE et al.** Attention-based Deep Multiple Instance Learning. *arXiv: 1802.04712v4 [cs.LG*